Europäisches Patentamt

European Patent Office

Office européen des brevets

(19)

(11) Publication number: **0 112 122 B1**

## (12) EUROPEAN PATENT SPECIFICATION

(45) Date of publication of patent specification: **28.08.91**

(51) Int. Cl.⁵: **C12N 9/72, A61K 37/54**

(21) Application number: **83307468.5**

(22) Date of filing: **08.12.83**

(54) Plasminogen activator.

(30) Priority: **14.12.82 ZA 829168**
        **11.01.83 AU 10296/83**

(43) Date of publication of application:
       **27.06.84 Bulletin 84/26**

(45) Publication of the grant of the patent:
       **28.08.91 Bulletin 91/35**

(84) Designated Contracting States:
       **CH DE FR GB LI SE**

(56) References cited:
       **EP-A- 0 023 860**
       **EP-A- 0 041 766**
       **EP-A- 0 113 319**
       **GB-A- 2 103 791**
       **US-A- 4 245 051**

       **BIOLOGICAL ABSTRACTS, RRM, ref.no.
       2516717, Philadelphia, US; C. HEUSSEN et
       al.:"An inhibitor of tissue plasminogen ac-
       tivator isolated from the seeds of the South
       African tree Erythrina-latissima"**

(73) Proprietor: **SOUTH AFRICAN INVENTIONS DE-
VELOPMENT CORPORATION
Administration Building Council for Scienti-
fic and Industrial Research Scientia Meiring-
naude Road
Pretoria Transvaal(ZA)**

(72) Inventor: **Dowdle, Eugene Bernard Davey
Annalong, 1 Norwich Drive Bishopscourt
Cape Town, 7700(ZA)**

(74) Representative: **Hardisty, David Robert et al
BOULT, WADE & TENNANT 27 Furnival Street
London EC4A IPQ(GB)**

## Description

The present invention relates to a pharmaceutical composition for the management or prophylaxis of thrombosis or embolism which contains a TPA/pro TPA coupled to the use of said couple in the manufacture of the pharmaceutical composition. The pro-TPA can be produced by a method which comprises cultivating a pro-TPA yielding culture in a suitable culture medium, followed by recovery of the pro-TPA (and optionally TPA).

The term TPA/pro-TPA couple as used herein is intended to denote either of or a combination of both of the tissue plasminogen activator defined further below and recoverable, e.g. from Bowes melanoma cell tissue cultures or its precursor pro-TPA also as defined further below.

TPA is the abbreviation adopted in the art for a tissue plasminogen activator, i.e. the 2-chain type of plasminogen activator which has been identified for example in Bowes melanoma cells. Pro-TPA is the abbreviated name given to the precursor of TPA, i.e. a single chain compound having the same molecular weight as TPA and which by certain enzymes is converted into double chain TPA. According to the prior art literature the single- and double-chain forms were said to both have virtually identical activity. The present invention will be shown to involve a radical departure from that view.

Plasminogen activators are substances which by their action upon plasminogen (a precursor of plasmin) result in the formation of plasmin. Plasmin in turn acts upon fibrin (or blood clots) to liquefy or dissolve the fibrin. Plasmin also causes lysis of fibrinogen which is a precursor of fibrin.

The aforegoing effects play an important role in the natural fibrinolytic systems. They are also put to use in the therapeutic administration of plasminogen activators for the management of thrombotic disease or other conditions where it is desirable to produce local fibrinolytic or proteolytic activity via the mechanism of plasminogen activation.

They may also find use in reagents for diagnostic, pathological or scientific tests involving fibrinolysis in vitro.

Two activators of human plasminogen are extensively used. The first of these is the bacterial protein, streptokinase, which function by a non-proteolytic mechanism. The second is the protease, urokinase, which is obtained from urine or cultured kidney cells, although it is also known to occur in most other kinds of mammalic tissue. These two compounds have the therapeutic disadvantage that their action is not confined to plasminogen associated with fibrin in blood clots. They act upon plasminogen generally in the circulation and, in consequence, produce widespread plasmin generation with extensive lysis of fibrinogen, the precursor of fibrin. This may in turn lead to a bleeding state as a result of ineffective normal coagulation of the blood. Furthermore, urokinase has had the disadvantage that it is costly to isolate and prepare and is obtained in poor yields by the processes currently in use. This disadvantage also applies to valuable uses of urokinase other than fibrinolytic therapy, and restricts the availability of urokinase as a valuable commercial product for such uses. Streptokinase,being a foreign protein, elicits an immune response from the patient. The resulting antibodies neutralise the action of streptokinase on plasminogen and hence diminish its therapeutic efficacy.

A third plasminogen activator referred to generally as "tissue plasminogen activator" hereinafter referred to as "TPA" is also known to exist. This enzyme is found in most human tissues and is identical or indistinguishable from an enzyme that is also a characteristic secretory product. of human melanoma cells cultured in vitro. Although it catalyses the proteolytic activation of plasminogen in much the same way as does urokinase, TPA differs from urokinase in a number of important respects. The two enzymes are chemically dissimilar. They have different molecular weights and each fails to react with antibodies to the other enzyme. The catalytic action of TPA is enhanced by fibrin whereas that of urokinase is not. TPA has the important property that it binds to fibrin, whereas urokinase fails to do so. These facts are recorded in a recent article entitled "Purification and characterisation of the plasminogen activator secreted by human melanoma cells in culture" by C Dingeman C. Rijken et alia (J. of Biological them., 256, No 13, pages 7035 to 7041). That same article also describes the preparation of TPA from a cultured human melanoma cell line as well as from normal human tissue. The purification procedure consisted of successive chromatography on zinc chelate-agarose, concanavalin A-agarose and sephadex G-150 in the presence of detergent.

The tendency for TPA to bind to fibrin, its greatly enhanced fibrinolytic action in the presence of fibrin and when compared to urokinase and streptokinase, its relatively inefficient function as a plasminogen activator in the absence of fibrin combine to make TPA a plasminogen activator of choice for human thrombolytic therapy. Interactions between fibrin and TPA to a considerable extent localise the plasmin generation to the site of the clot and mitigate or avoid the consequences of promiscuous plasminogen activation as observed when urokinase or streptokinase is used. Furthermore streptokinase is a protein which is immunochemically foreign to man, whereas TPA is not.

2

EP 0 112 122 B1

A recent report entitled "Specific lysis of an iliofemoral thrombis by administration of extrinsic (tissue type) plasminogen activator" by W. Weimar et alia (Lancet, November 7, 1981, page 1018) testifies to the clinical usefulness of TPA in the management of human thrombotic disease.

According to P. Wallen et al (Prog. Chem. Fibrinolysis Thrombolysis 5 , 16-23 (1981)) TPA occurs in two different forms, one being a single-chain form which is converted by plasmin or trypsin into two chains linked by bisulphide bridges, and which those authors consider to be a degradation product of the former. The two-chain form is said to be fibrinolytically more efficient in fibrinolytic test systems (in vitro) than the one-chain form. Aprotinin is said to inhibit the conversion of the one-chain into the two-chain form so that the one-chain form may be recovered as the only or predominating form. Both forms are considered to be tissue activators.

This work is referred to in more recent publications by Dingeman, Rijken, Hoylaerts and Collen (J. Biol. Chem., 257, 2920-2925 (1982), European patent application 0 041 766) who describe both forms as plasminogen activators, whose "plasminogen activating properties ... are similar". They suggest that the one chain form when it is adsorbed on a fibrin clot is quickly converted to the two-chain form so that the fibrinolysis "occurs mainly via two-chain derivative". They conclude: "this conversion does not seem to play a role in the regulation of fibrinolysis". According to their findings the "two molecular forms had the same activity (lysis time)", and "the catalytic efficiencies of both molecular forms are virtually identical therefore the one-chain form cannot be considered to be a less active precursor nor the two-chain form to be a less active degradation product". Whether or not the aforesaid conclusions are all correct, it is clear that both "molecular form" have utility for therapeutic as well as in vitro fibrinolysis.

In actual fact, in the light of recent research results by the present inventors, it is clear that the above conclusions require some qualification. Pro-TPA is indeed in a sense a precursor of TPA, although not a totally inactive precursor. Its inherent activity is about one tenth that of TPA, so that on that basis alone it would at first sight appear preferable to administer TPA for therapeutic purposes rather than pro-TPA which first has to be converted into TPA before it can exercise its full therapeutic effect.

As a matter of fact, although European patent application 0041766 describes two compounds which presumably represent TPA as well as pro-TPA, only the former compound is actually characterised in respect of its fibrinolytic, fibrinogenolytic and thrombolytic effects. There is no such description for pro-TPA, nor any specific description of its use as a pharmaceutical composition. The claims for the pharmaceutical use of the compounds, to the extent that they are specific are directed only to the double-banded form, i.e. TPA. The only published actual therapeutic use of the substance has been the use in the double-stranded TPA form (Weimer et al., The Lancet, Nov.7, 1981 p 1018 - 1020).

According to the aforesaid prior art, no method was known which lends itself readily to the manufacture of 2-chain TPA on a commercial scale, nor of the single chain form herein referred to as "pro-TPA".

According to a recent proposal by the present inventors (RSA patent application 82/9168 and applications based thereon in other countries and which at present are not yet part of the state of the art) a process has been proposed which lends itself readily to such commercial scale manufacture. This process provides a new and relatively simple process for the selective, reversible adsorption of TPA and/or pro-TPA from solutions, in particular for the manufacture of purified plasminogen activators.

The new process permits the manufacture optionally of purified TPA, but preferably purified pro-TPA and purified mixtures of TPA and pro-TPA wherein pro-TPA preferably predominate. The processes as aforesaid are capable of separating TPA and pro-TPA from urokinase and which if desired, can be adapted to the separate recovery of urokinase.

In particular it is an object to provide pharmaceutical preparations based on some not previously known properties and advantages of pro-TPA over TPA which can be put to beneficial use not only in the management of thrombosis and embolism, which have already occurred but also in the prophylaxis against such conditions.

The method of producing a purified plasminogen activator substance based on the TPA/pro-TPA couple (pro-TPA being as defined above) comprises cultivating a pro-TPA yielding cell culture in a suitable culture medium, wherein the cells are cultivated in the absence of enzymes which catalyse the conversion of pro-TPA into TPA or where such enzymes are present in the presence of a suitable inhibitor adapted to inhibit such enzymes, to produce a harvesting liquid wherein the couple is represented by 73 to 100% pro-TPA, the balance being TPA. The harvesting fluid containing the dissolved pro-TPA and contaminants is intimately contacted with an affinity reagent comprising an immobilised Kunitz type inhibitor of the type occurring in seeds of Erythrina Latissima and other Erythrina species and having the property of being an inhibitor for trypsin, plasmin and TPA, but having no effect on urokinase, thereby selectively adsorbing the pro-TPA and any TPA from the medium. The affinity reagent loaded with pro-TPA and any TPA is removed from contact with the aqueous medium and non-adsorbed contaminant. The pro-TPA and any TPA is

3

desorbed from the affinity reagent and the desorbed pro-TPA and optionally the TPA is recovered.

If a cell culture is used which also produces urokinase, the pro-TPA and any TPA in the harvesting fluid is contaminated with urokinase and the urokinase is removed with the non-adsorbed contaminants and can then optionally be recovered as a valuable product.

Preferably the aqeous medium, i.e. the harvesting fluid is derived from cultivating a melanoma cell culture.

The seed of Erythrina Latissima (broad-leafed Erythrina) and other Erythrina species contains two proteinase inhibitors (Francois J Joubert et alia, Z. Physiol. Chem. 362 pages 531 - 538). They are referred to in that publication as DE-1 and DE-3. Whereas DE-1 inhibits bovine chymotrypsin and not bovine trypsin, DE-3 inhibits trypsin but not chymotrypsin. One of these, DE-3, has the property of being an enzyme inhibitor of the Kunitz type and of being an inhibitor for trypsin, plasmin and TPA, but having no effect on urokinase. DE-3 was selected after screening hundreds of enzyme inhibitors for being the only one capable of distinguishing between TPA and urokinase. It was found that if this inhibitor is immobilised, e.g. in manners known per se, it forms an affinity reagent which selectively extracts by adsorption TPA - as well as the previously unknown precursor of TPA, pro-TPA, whilst leaving the contaminants unadsorbed in the aqueous solution. Because DE-3 is an inhibitor for plasmin, it prevents the conversion of pro-TPA into TPA by the action of the plasmin which is normally present in aqueous liquids suitable as sources of TPA and/or pro-TPA. Accordingly, this immobilised inhibitor has outstanding properties when used as an adsorbent for pro-TPA.A more complete chemical characterisation of DE-3 is contained in the above publication of Joubert el al.

Urokinase, if present in the aqueous solution is not adsorbed. Accordingly, the affinity reagent can also be used in the process according to the invention when the TPA and/or pro-TPA is contaminated with urokinase and the urokinase is removed with the non-adsorbed contaminant. This means that the process can be applied to recover pro-TPA and TPA from a large number of sources such as various kinds of tissue wherein TPA and/or pro-TPA occur in mixture with urokinase.

However, a particularly preferred aqueous liquid to be used for the extraction of pro-TPA and optionally TPA therefrom is a harvest fluid, preferably serum-free, obtained from a tissue culture of human melanoma cells containing pro-TPA and 0 -50% TPA in the secretory products of the cells.

The pro-TPA and any TPA is adsorbed by passing the aqueous medium containing it through a bed of the affinity reagent, followed by washing the bed and subsequent desorption of the pro-TPA and any TPA and washing the desorbed pro-TPA and any TPA off the bed. The bed may for example be in the form of a column.

The desorption is preferably carried out with aqueous potassium thiocyanate solution preferably from about 1 to 3 molar, e. g. from 1,4 to 2 molar, preferably 1,6 molar at a pH at which TPA is stable, preferably dissolved in approximately neutral phosphate buffered saline, e.g. between 0,3 and 1 molar, preferably about 0,4 molar in respect of NaCl and preferably containing a stabilising amount of a suitable surfactant, e.g. 0,1% Triton X-100 or Tween. The desorption may be carried out with chaotropic desorbents other than potassium thiocyanate. The desorption may also be carried out at low pH, e.g. from 2 to 3,5, preferably from 2,8 to 3. Other suitable desorbing conditions are high salt concentrations and/or the presence of arginine and/or the presence of benzamidine. Such desorbing conditions are known as such to persons skilled in the art and therefore require no detailed description.

Also, if it is desired to recover TPA free of pro-TPA, any pro-TPA, whether produced by the process steps described further above or not may be readily converted enzymatically into TPA, e.g. by exposing the pro-TPA in aqueous solution to the action of plasmin or enzymes having an equivalent effect on pro-TPA. Such an enzyme as yet unidentified occurs for example in fibrin for which reason the conversion of Pro-TPA into TPA also occurs in the presence of fibrin. Kallikrein also produces this effect albeit less effectively.

It has now been found that pro-TPA such as produced by the method described above differs from TPA in that the former is a single-chain compound which has only slight plasminogen activator properties, whereas TPA (molecular weight 72 000 dalton) is a double-chain compound and has pronounced plasminogen activator properties, about 10 times as strong as pro-TPA. Pro-TPA has the same molecular weight as TPA.

Because of this weak activity in the single-chain form and the knowledge that double-chain TPA is so much more active the prior art would normally have created a strong incentive to employ the plasminogen activator in pharmaceutical compositions in the TPA form (i.e. the double-stranded molecular form), because in vivo it is the TPA form which is predominantly effective in the clot dissolving process. This view is reinforced by the knowledge that pro-TPA itself is very weakly active.

However, surprisingly it has now been established by experimental evidence that there are convincing reasons for using the plasminogen activator in the pro-TPA form, preferably at least predominantly so but better entirely so, rather than using TPA as the active ingredient of pharmaceutical compositions for the management of thrombotic disease or other forms of therapy wherein use is made of the fibrinolytic effect of the plasminogen activator. The reason for this is that the pro-TPA has a higher affinity for adsorption to fibrin than the TPA form and that once it has been adsorbed by fibrin it is converted very rapidly into TPA and then acquires the full desired activity, exactly where it is needed, namely at the clot. The consequences to be concluded from this finding are

(a) that pro-TPA will be absorbed more rapidly and completely at a clotted fibrin site, that is where the substance is actually needed. There the pro-TPA is rapidly converted - in situ - into TPA by the proteolytic enzymes present in the fibrin whereupon the full plasminogen activating activity of TPA becomes available immediately at the actual site of the clot.

(b) Pro-TPA will be more selective than TPA for bringing about fibrinolysis because it is relatively inactive until it attaches to the fibrin and only becomes fully active by its conversion into TPA in situ. With TPA there exists an about 10 times greater probability that it may activate some plasminogen in the bloodstream instead of at the thrombus where the fibrinolytic effect is required. This relative probability is even further increased by the lower affinity of the TPA form for adsorption to the fibrin site.

(c) Because of the drastically reduced activity whilst in the bloodstream it can now be recommended with confidence to employ pro-TPA in prophylactic therapy, where there is a risk of future thrombotic or embolic occurrences.

(d) On the other hand the risk of side effects when using relatively high dosages in acute cases of thrombotic occlusions is also diminished.

(e) TPA has a short halflife in vivo. The properties of pro-TPA are such as to render the utilisation of the plasminogen activator more efficient . On the one hand pro⁼TPA is less likely to be consumed by ineffective reactions whilst still in the bloodstream. On the other hand it is more likely to be "captured" rapidly by the clot site, where it can be effective.

Based on the above important concept, in the light of the aforegoing, the invention provides a pharmaceutical composition for the management or prophylaxis of thrombosis or embolism which composition acts by means of local plasminogen activation and which is in dosage units for injection or intravenous infusion, said composition comprising

a) a TPA/pro TPA couple composed of 70 to 100% pro-TPA and up to 30% TPA and

b) a physiologically compatible medium.

Preferably the amount of TPA/pro TPA couple in said dosage units is such as to provide an average plasminogen activator activity in the bloodstream of less than half that which results from the administration of 15mg per 24 hours of TPA. As for the remainder, what has been stated above in connection with the method of treatment applies mutatis mutandi to the pharmaceutical composition.

The present invention is to be used for a method of fibrinolytic treatment for the management of prophylaxis of thrombosis, embolism or other conditions where it is desired to produce fibrinolytic or proteolytic activity via the mechanism of plasminogen activation, which comprises administering intravenously to a patient a plasminogen activator composition comprising a plasminogen activator component represented by the TPA/pro-TPA couple and composed of from 70 to 100 % pro-TPA and up to 30% TPA at a dosage rate designed to produce, in the bloodstream, an average plasminogen activator activity of less than half, e.g. between 5 and 30 % that which results from the administration of up to 15 mg per 24 hours of TPA.

Preferably the dosage of TPA/pro-TPA couple is up to about 15 g per 24 hours. Also the TPA/pro-TPA couple is represented by 70 to 100 % pro-TPA and up to 30 % TPA, more preferably by 80 to 100 % pro-TPA and up to 20 % TPA.

It is preferred (and readily possible because of the low activity of pro-TPA in therapeutically effective dosages) for the plasminogen activator activity in the bloodstream to be maintained at less than 20 % that which results from the administration of 15 mg per 24 hours of TPA.

The method may be applied to the management of thrombosis or embolism where thrombotic occlusion has already occurred and then comprises intravenously administering to the patient from 3 to 15 mg per 24 hours of the TPA/pro-TPA couple. This may be done in dosage units of 3 to 15 mg of TPA/pro-TPA couple. It may also be done by intravenous infusion at a rate of 1/8 to 5/8 mg per hour of TPA/pro-TPA couple.

The invention is also particularly applicable to the prophylaxis of conditions involving thrombotic occlusion comprising the intravenous administration to the patient of dosage units of from 1 to 10 mg of TPA/pro-TPA couple or the intravenous infusion of the plasminogen activator composition at a rate of 0,5 to 2 mg per hour of TPA/pro-TPA couple. Such prophylactic treatment may be administered to a patient showing no symptoms of thrombosis or embolisation at all but who is, nevertheless, at risk. Examples are bed-ridden patients or debilitated patients who, by virtue venous stasis are particularly liable to suffer deep vein thrombosis and embolisation. Other examples are patients with unstable angina in whom thrombotic coronary artery occlusion is likely to be imminent. Further examples are patients with cardiac valvular disease in which thrombine is situated in the legions and where there is a risk of cerebral embolisation. Examples are patients where the cavities of the heart or the great blood vessels have legions or damage such that a risk exists of blood clots becoming dislodged and causing cerebral vascular occlusion (strokes). Examples are also patients with prosthetic vascular valves and atrial fibrilation, sub-acute bacterial endocarditis etc. The method is also useful for the prophylactic treatment of patients receiving intravenous therapy or total parentheral nutrition, where the venous line is inserted into a large vein and where irritant solutions are infused, likely to cause venous thrombosis and occlusion and/or embolism.

Whilst continuous intravenous infusion is preferred for the administration, intermittent pulsed intravenous administration may be applied as well in the above dosages of e.g. 1 to 10 mg, say every 2 to 24 hours.

The present invention provides the various pharmaceutical compositions specially adapted and prepared for use in the above uses, and as more fully defined in the claims which are an integral part of the present disclosure.

The present invention allows the preparation of purified TPA or pro-TPA separately or as a mixture.

The present invention also permits the recovery of pro-TPA from an aqueous medium containing it and also containing plasmin and/or plasminogen and a plasminogen activator. This requires inhibiting plasmin in the aqueous medium and removing the pro-TPA from the aqueous medium whilst plasmin is inhibited. In this context it is pointed out that TPA, when present acts as a plasminogen activator causing plasmin to be formed, which in turn must be inhibited.

In the above-described process, the pro-TPA is adsorbed from the aqueous medium by an affinity reagent comprising an immobilised inhibitor for plasmin as well as having an affinity for pro-TPA, from which pr TPA is subsequently desorbed and recovered in purified form. The pro-TPA thus recovered may then be sterilised and stabilised with a physiologically compatible stabiliser in the form of an intravenously injectable aqueous solution.

Because the DE-3 inhibitor is used in its immobilised form as an affinity reagent happens to be an inhibitor of plasmin and because plasmin is normally present in the media from which the TPA and pro-TPA is to be recovered and has the property of converting pro-TPA into TPA, the use of this inhibitor to isolate pro-TPA simultaneously serves to preserve the pro-TPA in its pro-TPA form.

It is possible to selectively adsorb TPA and/or pro-TPA separately from and in addition to urokinase from aqueous media containing both plasminogen activators (as such or as a precursor). This may be applied to the manufacture of either purified TPA and/or pro-TPA free of urokinase on the one hand or of purified urokinose free of TPA and pro-TPA on the other hand or of both purified types of plasminogen activators. This can be done by contacting such an aqueous medium with an affinity reagent comprising an immobilised Kunitz type inhibitor of the type occurring in seeds of Erythrina latissima and other Erythrina species and having the property of being an inhibitor for trypsin, plasmin and TPA, but having no effect on urokinase, thereby selectively adsorbing the TPA and/or pro-TPA from the solution, removing the affinity reagent loaded with TPA and/or pro-TPA from contact with the aqueous medium depleted of TPA and/or pro-TPA, but still containing the urokinase, and recovering urokinase from the aquous medium depleted of TPA and/or pro-TPA. Suitable, aqueous media for this process will be aqueous extracts of random mammalian tissue cells or media containing the secretion products of such cells since such media contain both plasminogen activators. Once again, the adsorbed TPA and/or pro-TPA may be desorbed and recovered and if desired pro-TPA may be converted into TPA either before or after the adsorption on the selective affinity reagent.

The urokinase may be recovered from the aqueous medium in any suitable manner, for example by being adsorbed from the aqueous medium using an affinity reagent having an affinity for urokinase. Such affinity reagents may be immobilised inhibitors having no selectivity for TPA over urokinase. It is also possible to use as an affinity reagent antibodies against urokinase, suitably immobilised in a manner known per se.

For use in the process the Kunitz type inhibitor of the type occurring in seeds of Erythrina latissima and other Erythrina species and having the property of being an inhibitor for trypsin, plasmin and TPA, but having no effect on urokinase, is immobilised by being bonded to a solid carrier, e.g. by being covalently bonded to the carrier For example, the inhibitor may be coupled to cyanogen bromide activated agarose in a manner known as such to persons skilled in the art. However, other solid carriers can be used and other coupling agents depending on the chemical properties of the surface to which the inhibitor is to be coupled. Examples are

(a) carbodiimide coupling which couples free amino groups to free carboxy groups;

(b) glutaraldehyde coupling which couples NH$_2$ groups to agarose previously converted into the amino-alkyl form;

(c) periodate activation of agarose to generate aldehyde functions which are subsequently reacted at pH 4 to 6 with amines of the inhibitor to form Schiff bases which are then reduced with sodium borohydride or sodium cyano borohydride;

(d) the agarose may be converted into the hydrazidosuccinyl derivative; to this carboxylic ligands may be attached with EDC, or amines by diazotisation;

(e) cellulose fibres or beads may be converted into a hydrazide derivative and used according to the method of Parikh et alia (Methods in enzymology Vol. 34, pages 77 to 102, editors W B Jakobi and Wilcheck, Academic Press NY).

(f) polyacryl amide beads may be coupled by direct glutaraldehyde procedure or by diazotisation of the p-aminobenzamido ethyl derivatives or of the hydrazido derivative;

(g) proteins, e.g. albumin or trypsin or gelatin may be coupled, e.g. in the form of hydrazides;

(h) finally, reference is made to glass beads (or other solid bodies) which can be coated with a protein, e.g. gelatin which may be cross linked and coupled to the inhibitor by methods analogous to any of the coupling methods described in RSA patent application No. 81/4481, and corresponding applications abroad (German patent application P 3224837.7, Japanese patent application 116086/1982, United Kingdom patent application 8219070 and USA patent application 392111).

The aforegoing methods are known as such to persons skilled in the art and/or are described in the cited literature and require no detailed description.

The affinity reagent may be used for the manufacture of TPA and/or pro-TPA and of preparations comprising TPA and/or pro-TPA, e.g. in the processes as set out further above. However, the affinity reagent can also have utility as a diagnostic or analytical reagent.

In order to produce even higher ratios, e.g. as high as 9:1 or more, it is proposed to produce the pro-TPA by cultivation of pro-TPA-yielding cell cultures, e.g. melanoma cells in a suitable culture medium, either in the absence of enzymes which catalyse the conversion of pro-TPA to TPA or in a medium containing such enzymes, e.g. containing serum, and suitable inhibitors adapted to inhibit such enzymes (more particularly plasmin) which catalyse the conversion of pro-TPA to TPA. Suitable inhibitors are Soyabean-derived trypsin inhibitors or alpha I-antiplasmin. Aprotinin can be used instead (as is already known). The pro-TPA is then recovered from the medium in the absence of said enzymes or in the presence of the inhibitor(s).

If a mixture of pro-TPA and TPA is available and if it is desired to restrict the enzymatic activity thereof to that of pro-TPA, it is possible to remove the TPA by using an inhibitor which irreversibly inhibits TPA. A suitable example thereof is the irreversible proteinase inhibitor diisopropyl phosphofluoridate. The inhibitor may subsequently be removed by dialysis or gel chromatography. The irreversibly inhibited TPA may be left in the mixture.

The pharmaceutical compositions comprising TPA and/or pro-TPA prepared or purified by the aforementioned method may be dissolved in a physiologically compatible injection medium. The claimed composition may also be used as fibrinolytic preparations for carrying out fibrinolysis in vitro, e.g. for diagnostic or pathological or general scientific purposes.

It is considered an advantage of the aforementioned method that it can be adapted substantially at will to produce a couple as aforesaid comprising optionally either TPA or pro-TPA as the only active ingredient or TPA and pro-TPA in a desired ratio.

The invention will now be described by way of example, more particularly by way of the following non-limiting embodiment(s), which, if read in the context of the aforegoing general description, will enable the person skilled in the art to practise the full scope of the invention.

Example 1

Preparation of affinity reagent according to the invention

DE-3 inhibitor is prepared as follows :
Erythrina latissima seeds are collected and processed according to the method of Joubert et alia. The seeds are ground, defatted and extracted at 10 °C overnight with 0,5 molar sodium chloride solution. The extract is centrifuged and DE-3 inhibitor is recovered from the supernatant by ammonium sulphate precipitation followed by chromatography on Sephadex G50, DEA-cellulose and DEA-sepharose. The finally purified material migrates as a single band with an apparent molecular weight of 22 000 daltons when subjected to electrophoresis on a 15% polyacryl amide gel containing 0,1% sodium dodecyl sulphate (SDS).

Purified DE-3 (26 mg) is coupled to 5 ml of commercially available cyanogen bromide activated agarose in the usual manner (Sepharose 4b marketed by Pharmacia and who also supply the instructions for the use of the preparation). The affinity reagent was equilibrated against phosphate buffered saline of pH 7,4 containing 0,4 M sodium chloride, 0,1% Triton X-100 (a commercial detergent commonly used in this art) and 0,02% sodium azide as a stabiliser. This affinity reagent is then packed into a 5 ml column fashioned from the barrel of a disposable plastics syringe.

Example 2

Preparation and purification of TPA and pro-TPA

A medium containing TPA and pro-TPA is produced as follows: Human melanoma cell of a cell line known as Bowes cells RPMI-7272 (obtained from Denver Hospital, Denver, Colorado and described in J.Biol.Chem 256, 7035-7041) are grown as adherent monolayers in RPMI 1640 tissue culture medium supplemented with 10% heat inactivated (56 °C; 30 minutes) foetal bovine serum (FCS) and antibiotics (300 $\mu$ g per ml penicilin; 200 $\mu$ g per ml streptomycin and 10 $\mu$ g per ml of tylocine). The cells are passaged at confluence (approximately 4 x $10^5$ cells per cm$^2$ ) by trypsinisation and reseeding at 5 x $10^5$ cells per 100 mm dish. After aspirating the medium, the cells are incubated in 0,25% trypsin in tris dulbecco's saline (24,8 mM tris HCl pH 7,4 containing 0,1 mM Na$_2$HPO$_4$, 5 mM KCl, 0,14 M NaCl) at 37 °C for 5 minutes. Detached cells are dispersed by gentle pipetting, and the suspension is added to an equal volume of medium containing foetal bovine serum to neutralise the protease. The cells are then washed by centrifugation at 350 g for 5 minutes, resuspended and reseeded into fresh dishes.

From these stock cultures the cells are grown as adherent monolayers in 75 cm$^2$ or 150 cm$^2$ tissue culture flasks and allowed to grow to near confluency in mediums supplemented with 10% foetal bovine serum. In order to raise the eventual ratio of pro-TPA : TPA above 1:1 the proteolytic enzymes (i.e. plasmin) in the serum are removed or inhibited with aprotinin or as more fully described in Example 5. The cultures are then washed once and covered with 20 ml of serum-free medium. After 24 hours the medium is collected and fresh medium is added.

The harvest fluids are centrifuged at 2000 rpm for 5 minutes to remove whole cells and cellular debris. The solution is stabilised with Triton X100 (0,1%) or Tween 80 (0,1%) and acidified with glacial acetic acid (pH 5,5 to 6,0) for storage at -20 °C.

2$\ell$ of harvest fluid are made 0,4 molar with respect to NaCl and are filtered through a 0,45 $\mu$ m membrane. The filter harvest fluid is then applied to the affinity column at a flow rate of 45 ml/hour at room temperature. The effluent is collected at 4 °C and monitored for the presence of plasminogen-dependent fibrinolytic activity. No TPA or pro-TPA is detected.

After the total volume of harvest fluid has passed through the column is washed with 6 column volumes of phosphate buffered saline containing 0,4 M NaCl and 0,1% Triton X-100. No TPA or pro-TPA is eluted by this procedure.

Adsorbed proteins are then eluted using PBS containing 1,6 M potassium thiocyanate, 0,4 M NaCl and 0,1% Triton X-100.

The protein content is monitored by reading the absorbance at 280 nm. All plasminogen-dependent fibrinolytic activity is found to be eluted as a sharp peak when potassium thiocyanate is added to the eluting buffer, and this peak corresponds to a small protein peak.

The fractions containing the highest activity are pooled to give 6 to 8 ml solution stored at -20 °C and which represents 70 to 80% of the activity applied to the column. Fractions containing low activity are pooled separately. The total recovery of activity in both pools usually amounts to 90 to 100%. The potassium thiocyanate eluate contains a single band of protein with a molecular weight of 72 000 daltons corresponding to a mixture of pure TPA and pro-TPA, the latter in different runs amounting to from 39 to about 50% of the total or more.

The yields improve if the same column is used repeatedly over and over again. The column thus not only remains effective to be re-used many times, but in fact improves with use. This is surprising because TPA has the property of cleaving the inhibitor DE-3 when in its non-immobilised form.

Example 3

Pharmaceutical preparation containing TPA and/or pro-TPA

The pooled fraction containing TPA and/or pro-TPA are dialysed against 0,30 molar sodium chloride containing 0,01 volume percent Tween 80 and stored at -80 °C. Prior to administration the concentration is adjusted to 75 $\mu$ g per ml of TPA, plus pro-TPA (for therapeutic purposes the dosage rate for TPA is the same as for pro-TPA) and 0,3 molar NaCl. The solution is sterilised by filtration through an 0,22 $\mu$ m membrane filter.

The solution is administered intravenously by infusion at a dosage rate of between 3 and 15 mg per 24 hours, typically 7,5 mg per 24 hours. Ideally the dosage rate should be such that during the treatment the total plasminogen activator (including precursor) level in the blood rises to between 0,6 and 1, preferably about 0,8 international units per ml. At such concentrations the treatment is found to be highly effective for clearing thrombosis and no systemic fibrinolysis is observed.

However, because pro-TPA is only converted to TPA after binding to fibrin has occurred, the actual activity whilst still in the blood stream is found to be a small fraction, as little as 10% of that which would be present if all the plasminogen activator were to be in the fully active TPA form. Because moreover pro-TPA is more quickly and more strongly adsorbed by fibrin than is TPA. The reaction of promiscuous reactions is reduced even further.

## Example 4.

### Mass cultivation of melanoma cells

A spinner flask charged with Rosswell Park Memorial Institute culture medium 1640/10% foetal bovine serum is inoculated with melanoma cells at a concentration of $5 \times 10^5$ cells per ml. The flask is kept at 37 °C and stirred magnetically at 30 rpm. Medium is pumped out of the flask at a rate of 1% the volume of the flask per hour and replaced at the same rate with fresh medium which has been equilibrated with 5% $CO_2$ in air. The cells are retained in the culture vessel by an outlet glass tube plugged with glass wool. The withdrawn medium is filtered through a millipore filter.

The collected liquid is pasteurised and can now be processed according to Example 2.

## Example 5

### Cultivation of Melanoma cells for high pro-TPA yields

Example 2 is modified by the addition to the culture medium of sufficient soybean trypsin inhibitor or alpha-1-antiplasmin to inhibit the serum plasmin. In all other respects the process is carried out as in Example 2, the ratio of pro-TPA to TPA in the final product being now increased to approximately 9:1. The experimental details were as follows:

The Bowes cell culture medium contained 0,1% triton and 10 KIU trasylol (plasmin inhibitor). The initial harvesting fluid had a volume of 1960 ml. The KSCN eluate had a volume of 20,8 ml. The recovery of TPA + pro-TPA was 100% of which 87% was in the pro-TPA form. In a comparative experiment carried out without trasylol the recovery was 98%, composed entirely of TPA and no detectable pro-TPA.

## Example 6

### Elimination of residual TPA in mixtures of pro-TPA and TPA

The solution of TPA and pro-TPA is rendered 10 mM with respect to diisopropyl phosphofluoridate. The pH is adjusted to 8. After 4 hours the TPA has been permanently inhibited. The residual inhibitor is thereafter removed by dialysis.

## Example 7

### Conversion of pro-TPA into TPA

An aqueous solution containing 100 $\mu$ g of protein/per ml is mixed with an equal volume of phosphate buffered saline containing 5 $\mu$ g/per ml of plasmin and is incubated for 16 hours at 20 °C. Sodium dodecyl sulphate is added to a final concentration of 0,1% and the proteins are precipitated with 6% trichloracetic acid. The precipitate is washed in acetone and redissolved in 0,06 M Tris HCl pH 6,8 containing 1% sodium dodecyl sulphate and 10% glycerol.

After this treatment it can be shown by electrophoresis that the pro-TPA has been converted completely into the active TPA form.

The claims which follow are to be considered an integral part of this disclosure.

Example 8. The selectivity of pro-TPA for insolubilised fibrin

Samples of TPA/pro-TPA couple containing different percentages of pro-TPA (the balance being TPA were tested under identical conditions to establish the relative proportions of substance bound to a sample of fibrin in each case.

The percentages of pro-TPA in the sample (the balance being TPA were determined by measuring the fibrinolytic activities of the samples before and after treatment with plasmin (in order to convert pro-TPA into TPA). Thereafter a 10% correction factor was applied to compensate for the fact that pro-TPA has 10% of the fibrinolytic activity of TPA. The samples were then diluted in 0,1 molar tris HCl, pH 8.1, containing 0.1% Triton-X- 100 so that 0,2 ml would in the presence of micrograms of plasminogen release approximately 30 to 50% of fibrin, labelled with iodine 125 isotope coated on the bottom of Limbro wells in the course of one hour. Aliquots (0,2 ml ) were added to Limbrowells coated with the radioactive iodine labelled fibrin in quadruplicate. After incubation for one hour at 0 °C two wells of each quadruplicate set were treated three times with the Tris HCl to remove unbound TPA. Thereafter the bound activity was determined. These results were compared with results obtained on wells that had not been washed. The results are summarised in the following table.

| % activity | % pro-TPA (corrected) | Fibrinolytic activity ($c\%$ $^{125}$I-fibrin solubilised in 60 min) | | |
|---|---|---|---|---|
| | | Total activity | Activity after wash | % bound |
| 88.7 | 97.6 | 29.83 | 29.48 | 98.8 |
| 34.0 | 37.4 | 45.90 | 37.52 | 81.7 |
| 64.0 | 70.4 | 56.56 | 41.53 | 73.4 |
| 87.0 | 95.7 | 48.88 | 48.76 | 99.8 |
| 88.0 | 96.8 | 53.85 | 52.49 | 97.5 |

The results demonstrate that pro-TPA is substantially more completely bound by the fibrin than TPA.

Example 9. Prophylactic Treatment

Prophylactic treatment is given to patients for whom a substantial risk of thrombosis or embolisation exists (note: the treatment is contra-indicated for post operative treatment or immediately post partem). Suitable patients include bed-ridden or debilitated patients who, by virtue of venous stasis are prone to deep vein thrombosis and embolisation, patients with unstable angina in whom thrombotic coronary artery occlusion is thought to be imminent, patients with diseased heart cavities or great blood vessels,prosthetic vascular valves, atrial fibrillation, sub-acute bacterial andocarditis, patients receiving intravenous treatment with irritant solutions.

Example 10

It was determined that the plasminogen activating activity of pro-TPA is not zero as in most enzyme precursors, but that it amounts to 10% that which is obtained after complete conversion into double-chain TPA.

The effect is that the administration of TPA/pro-TPA couple comprising 50 - 100% pro-TPA and 0 - 50% TPA (even at the time of injection when the concentrations are highest, having regard to the half life of the substances in vivo) results in a much lower activity in the bloodstream than would arise from the administration from an equivalent amount of TPA. This will be understood from the following table :

| percentage TPA % | pro-TPA % | dosage | % activity in dosage compared with 15 mg pure TPA |
|---|---|---|---|
| 50 | 50 | 4 mg | 14,7% |
|  |  | 8 mg | 29,3% |
|  |  | 10 mg | 36,7% |
|  |  | 13 mg | 47,79% |
| 30 | 70 | 4 mg | 8,7% |
|  |  | 8 mg | 19,7% |
|  |  | 15 mg | 37 % |
| 20 | 80 | 4 mg | 7,5% |
|  |  | 8 mg | 14,9% |
|  |  | 15 mg | 28 % |
|  |  | 20 mg | 37,3% |
|  |  | 25 mg | 46,7% |
| 0 | 100 | 4 mg | 2,6% |
|  |  | 15 mg | 10 % |
|  |  | 25 mg | 16,7% |
|  |  | 50 mg | 33,3% |

The above table demonstrates the reduced probability of promiscuous plasminogen activation in the bloodstream resulting only from the newly discovered fact that pro-TPA has only 10% the activity of TPA. Added to this is the newly discovered effect of pro-TPA being adsorbed more selectivity (i.e. rapidly and strongly) from the bloodstream as soon as it reaches a fibrin site (see Example 8).

For this kind of treatment the dosage rate is 1 to 10 mg by intermittent pulse intravenous administration once every 2 to 24 hours. For this purpose ampoules are made up containing the desired amount of from 1 to 10 mg dissolved in 5 ml 0,30 molar saline containing 0,01 volume percent Tween 80 and optionally conventional concentrations of stabilisers such as albumin or manetol.

Infusion solutions are made up in the same medium in concentrations designed for an administration rate of 0,5 - 2 mg per hour of pro-TPA/TPA couple.

A typical such solution would contain between 4 mg per liter (tower range)and up to 16 mg per liter (higher range) or 8 mg per liter (average).

## Claims

1. A pharmaceutical composition for the management or prophylaxis of thrombosis or embolism which composition acts by means of local plasminogen activation and which is in dosage units for injection or intravenous infusion, said composition comprising:-
    a) a TPA/pro-TPA couple composed of 70% to 100% pro-TPA and up to 30% TPA, and

b) a physiologically compatible medium.

2. The composition of claim 1 wherein the amount of the TPA/pro-TPA couple in said dosage units is such as to provide an average plasminogen activator activity in the bloodstream of less than half that which results from the administration of 15 mg per 24 hours of TPA.

3. The composition of claim 1 or 2 in dosage units of 3 - 15 mg of the TPA/pro-TPA couple, for a 24 hour treatment.

4. The composition of claim 1 or 2 adapted for intravenous infusion and formulated for a dosage rate of 3 - 15 mg/24 hours (1/8 - 5/8 mg/hr).

5. The composition of any one of claims 1 to 4 characterised in that the TPA/pro-TPA couple is represented by essentially pure pro-TPA.

6. The composition of claim 1 for the prophylaxis of conditions likely to result in thrombosis or embolism in dosage units for intravenous administrations of 1 to 10 mg of TPA/pro-TPA couple or in the form of an infusion solution adapted for an infusion rate of 0.5 to 2 mg per hour of TPA/pro-TPA couple.

7. The composition of any one of claims 1 to 6 characterised in that the TPA/pro-TPA couple has been purified by a process which comprises intimately contacting an aqueous medium containing dissolved pro-TPA and optionally TPA with an affinity reagent comprising an immobilised Kunitz type inhibitor of the type occurring in seeds of Erythrina latissima and other Erythrina species and having the property of being an inhibitor for trypsin, plasmin and TPA, but having no effect on urokinase, thereby selectively adsorbing the pro-TPA and/any TPA from the solution, removing the affinity reagent loaded with pro-TPA and/any TPA from contact with the aqueous medium depleted of pro-TPA and/any TPA, and recovering the pro-TPA and/optionally TPA.

8. The composition of any one of claims 2 to 7, characterised in that the concentration of the TPA/pro-TPA couple in the dosage units is such as to produce on injection into the bloodstream an average plaminogen activator activity of 5 to 30% that which results from the administration of up to 15 mg per 24 hours of TPA.

9. The composition of any one of claims 2 to 7, characterised in that the concentration of the TPA/pro-TPA couple in the dosage units is such as to produce on injection into the bloodstream an average plasminogen activator of less than 20% that which results from the administration of up to 15 mg per 24 hours of TPA.

10. The use of the TPA/pro-TPA couple composed of from 70% to 100% pro-TPA and up to 30% TPA in the preparation of a pharmaceutical composition as claimed in claim 1 for use as a local fibrinolytic agent in the treatment or prophylaxis of thrombosis embolism and the like.

**Revendications**

1. Composition pharmaceutique pour le traitement ou la prophylaxie de la trombose ou de l'embolie, composition qui agit au moyen de l'activation de plasminogène locale et qui se présente sous forme posologique unitaire pour l'injection ou perfusion intraveineuse, composition comprenant :
   a) couple TPA(activateur du plasminogène tissulaire)/pro-TPA(précurseur de l'activateur du plasminogène tissulaire) constitué par 70 % à 100 % de pro-TPA et jusqu'à 30 % de TPA, et
   b) un milieu physiologiquement compatible.

2. Composition selon la revendication 1, dans laquelle la quantité du couple TPA/pro-TPA dans des formes posologiques unitaires est telle à fournir une activité moyenne d'activateur de plasminogène dans le circuit sanguin inférieure à la moitié de celle qui se produirait avec l'administration de 15 mg pour 24 heures de TPA.

3. Composition selon la revendication 1 ou 2, dans des formes posologiques unitaires de 3-15 mg du couple TPA/pro-TPA, pour un traitement de 24 heures.

4. Composition selon la revendication 1 ou 2, apte à la perfusion intraveineuse et formulée pour un débit posologique de 3-15 mg/24 heures (1/8 - 5/8 mg/h).

5. Composition selon l'une quelconque des revendications 1 à 4, caractérisée en ce que le couple TPA/pro-TPA est représenté par de pro-TPA essentiellement pur.

6. Composition selon la revendication 1 pour la prophylaxie de conditions susceptibles d'entraîner une trombose ou une embolie, dans des formes posologiques unitaires pour les administrations intraveineuses de I à 10 mg du couple TPA/pro-TPA ou sous forme d'une solution de perfusion adaptée pour un débit de perfusion de 0,5 à 2 mg par heure du couple TPA/pro-TPA.

7. Composition selon l'une quelconque des revendications 1 à 6, caractérisée en ce que le couple TPA/pro-TPA a été purifié par un procédé qui consiste à mettre intimement en contact un milieu aqueux contenant du pro-TPA dissous et facultativement du TPA ayant un réactif d'affinité comprenant un inhibiteur de type Kunitz immobilisé tel que celui se présentant dans les graines d'érythrina latissima et autres espèces d'érythrina et dont la propriété est d'être un inhibiteur pour la trypsine, la plasmine et le TPA mais n'ayant aucun effet sur l'urokinase, permettant ainsi d'adsorber sélectivement le pro-TPA et/ou tout TPA de la solution, de soustraire le réactif d'affinité chargé de pro-TPA et/ou de tout TPA du contact avec le milieu aqueux débarrassé du pro-TPA et/ou de tout TPA, et de récupérer le pro-TPA et/ou facultativement le TPA.

8. Composition selon l'une quelconque des revendications 2 à 7, caractérisée en ce que la concentration du couple TPA/pro-TPA dans des formes posologiques unitaires est telle à produire lors de l'injection dans le circuit sanguin une activité moyenne d'activateur de plasminogène de 5 à 30 % de celle qui se produirait avec l'administration jusqu'à 15 mg pour 24 heures de TPA.

9. Composition selon l'une quelconque des revendications 2 à 7, caractérisée en ce que la concentration du couple TPA/pro-TPA dans des formes posologiques unitaires est telle à produire lors d'une injection dans le circuit sanguin une activité d'activateur de plasminogène inférieure à 20 % de celle qui se produirait avec l'administration jusqu'à 15 mg pour 24 heures de TPA.

10. Utilisation du couple TPA/pro-TPA composé de 70 % jusqu'à 100 % de pro-TPA et jusqu'à 30 % de TPA dans la préparation d'une composition pharmaceutique selon la revendication 1 pour l'utilisation en tant qu'agent fibrinolytique local dans le traitement ou la prophylaxie de la trombose, de l'embolie et d'infections similaires.

**Patentansprüche**

1. Pharmazeutische Zusammensetzung zur Behandlung oder Prophylaxe von Thrombose oder Embolie, die durch eine örtliche Plasminogenaktivierung bewirkt wird und die in Dosiereinheiten zur Injektion oder zur intravenösen Infusion vorgesehen ist, wobei die Zusammensetzung folgendes enthält:
   a) ein TPA/Pro-TPA-Paar (Gewebe-Plasminogenaktivator/Pro-Gewebe-Plasminogenaktivator-Paar), zusammengesetzt aus 70% bis 100% Pro-TPA und bis zu 30% TPA, und
   b) ein physiologisch verträgliches Medium.

2. Zusammensetzung nach Anspruch 1,
   **dadurch gekennzeichnet,**
   daß der Betrag des TPA/Pro-TAP-Paars in den Dosiereinheiten ein solcher ist, daß eine durchschnittliche Plasminogenaktivator-Tätigkeit im Blutstrom von weniger als der Hälfte davon vorgesehen ist, was sich durch die Verabreichung von 15 mg TPA pro 24 Stunden ergibt.

3. Zusammensetzung nach Anspruch 1 oder 2,
   **dadurch gekennzeichnet,**
   daß Dosierungseinheiten von 3 - 15 mg TPA/Pro-TPA-Paar für eine Behandlung während 24 Stunden vorgesehen ist.

4. Zusammensetzung nach Anspruch 1 oder 2,
   **dadurch gekennzeichnet,**

daß sie für eine intravenöse Infusion eingerichtet ist und für eine Dosierungsrate von 3 - 15 mg pro 24 Stunden (1/8 - 5/8 mg/Std.) angesetzt ist.

5. Zusammensetzung nach einem der Ansprüche 1 bis 4,
   **dadurch gekennzeichnet,**
   daß das TPA/Pro-TPA-Paar im wesentlichen durch reines Pro-TPA vertreten ist.

6. Zusammensetzung nach Anspruch 1 für die Prophylaxe von Bedingungen, die möglicherweise zu Thrombose oder Embolie führen, und zwar in Dosiereinheiten für intravenöse Verabreichungen von 1 bis 10 mg von TPA/Pro-TPA-Paar in Form einer Infusionslösung für eine Infusionsrate von 0,5 bis 2 mg/Std von TPA/Pro-TPA-Paar.

7. Zusammensetzung nach einem der Ansprüche 1 bis 6,
   **dadurch gekennzeichnet,**
   daß das TPA/Pro-TPA-Paar durch ein Verfahren gereinigt wird, das einen engen Kontakt mit einem wäßrigen Medium einschließt, das aufgelöstes Pro-TPA und wahlweise TPA mit einem Affinitätsreagens enthält, das einen immobilisierten Kunitz Inhibitor des Typs einschließt, der die in Samen von Erythrine latissima und anderen Erythrinearten auftritt und die Eigenschaft besitzt, ein Inhibitor für Trypsin, Plasmin und TPA zu sein, jedoch keine Wirkung auf Urokinase hat, wodurch wahlweise das Pro-TPA und/jedes TPA aus der Lösung absorbiert wird, und das Affinitätsreagens, das mit Pro-TPA und/jedem TPA beladen ist, vom Kontakt mit dem wäßrigen Medium entfernt wird, das von Pro-TPA und/jedem TPA entleert ist und das Pro-TPA und/jedes TPA wiedergewonnen wird.

8. Zusammensetzung nach einem der Ansprüche 2 bis 7,
   **dadurch gekennzeichnet,**
   daß die Konzentration des TPA/Pro-TPA-Paares in den Dosiereinheiten derart ist, daß sie bei einer Injektion in den Blutstrom eine durchschnittliche Plasminogenaktivatortätigkeit von 5% bis 30% entwikkelt, was durch die Verabreichung von bis zu 15 mg TPA pro 24 Stunden erzielt wird.

9. Zusammensetzung nach einem der Ansprüche 2 bis 7,
   **dadurch gekennzeichnet,**
   daß in der Konzentration des TPA/Pro-TPA-Paares solche Dosiereinheiten sind, daß bei Injektion in den Blutstrom ein durchschnittlicher Plasminogenaktivator von weniger als 20% erstellt wird, was durch die Verabreichung von bis zu 15 mg TPA pro 24 Stunden erzielt wird.

10. Verwendung von TPA/Pro-TPA-Paar, bestehend aus 70% bis 100% Pro-TPA und bis zu 30% TPA bei der Herstellung einer pharmazeutischen Zusammensetzung nach Anspruch 1 zur Verwendung als örtliches, fibrinlösendes Mittel bei der Behandlung oder Prophylaxe von Thrombose, Embolie oder dergleichen.

14